# EUROPÄISCHE PATENTSCHRIFT

(11) **EP 3 107 410 B1**
(45) Veröffentlichungstag und Bekanntmachung des Hinweises auf die Patenterteilung: **19.04.2017**
(21) Anmeldenummer: 15817089.4
(22) Anmeldetag: 01.12.2015
(51) Int. Cl.: A41H 42/00, A41H 43/02, A41D 1/06, A41B 9/00, A41D 31/02

(54) **VERFAHREN ZUR HERSTELLUNG ELASTISCHER HOSEN**
METHOD FOR MANUFACTURING ELASTIC PANTS
PROCÉDÉ DE FABRICATION DE CULOTTES EN TISSU ÉLASTIQUE

(30) Priorität: 04.12.2014 DE 102014117935
(43) Veröffentlichungstag der Anmeldung: 28.12.2016
(73) Patentinhaber: Elastec Suisse AG, 8820 Waedenswil (CH)
(72) Erfinder: FENSKE, Wilfried, 8820 Waedenswil (CH); FENSKE, Sandra, 8820 Waedenswil (CH)
(74) Vertreter: Katscher Habermann Patentanwälte
(86) Internationale Anmeldenummer: PCT/EP2015/078210
(87) Internationale Veröffentlichungsnummer: WO 2016/087440

(56) Entgegenhaltungen:
- WO-A1-2005/115754
- DE-A1-102010 013 288
- GB-A- 1 250 788

## Beschreibung

Die Erfindung betrifft ein Verfahren zur kontinuierlichen Herstellung elastischer Hosen, wobei ein flächiges Bahnmaterial in einer Längsrichtung des Bahnmaterials fortlaufend transportiert, zu einem geschlossenen Schlauch geformt und in Längsrichtung verlaufend verschlossen wird, wobei der verschlossene Schlauch eine erste äußere Schicht bildet und wobei die erste äußere Schicht in Transportrichtung nachfolgend mit elastischen Fäden in Form einer Schlaucharmierung umwickelt wird, sodass die elastischen Fäden gespannt sind, wenn die äußere Schicht eine konvexe Form aufweist, wobei auf die elastischen Fäden in Transportrichtung nachfolgend fortlaufend eine zweite äußere Schicht aufgebracht und mit den elastischen Fäden und/oder der ersten äußeren Schicht verbunden wird, wobei die erste äußere Schicht, die elastischen Fäden und die zweite äußere Schicht ein elastisches Hosenbahnmaterial .

Aus dem Stand der Technik ist es bekannt, elastische Hosen und insbesondere elastische Unterbekleidung aus flächenelastischen Materialien herzustellen. Durch Verwendung solcher flächenelastischer Materialien wird der Tragekomfort der üblicherweise hauteng anliegenden Unterbekleidung deutlich verbessert, da ein guter Sitz der Unterbekleidung bei einem vergleichsweise geringen auf den Körper wirkenden Flächendruck erreicht wird, da die für den erforderlichen Halt und einen guten Sitz der Unterbekleidung erforderliche kraftschlüssige Verbindung mit der Haut über die gesamte Anlagefläche der Unterbekleidung an der Haut erreicht wird.

Ein Verfahren zur Herstellung eines solchen flächenelastischen textilen Materials, das insbesondere zur Herstellung von Unterbekleidung verwendet werden kann, ist in der Druckschrift EP 1 753 618 B1 beschrieben. Bei diesem Verfahren wird ein flächiges Bahnmaterial in einer Längsrichtung des Bahnmaterials fortlaufend transportiert und über einen langgestreckten Stab- oder rohrartigen Formkern in eine konvexe, schlauchartig geschlossene Form gefalten. Der auf diese Weise gebildete Schlauch bildet eine erste äußere Schicht auf dessen Außenfläche elastische Fäden in Form einer Schlaucharmierung aufgebracht werden, wobei die elastischen Fäden beim Aufbringen auf die erste äußere Schicht unter einer Vorspannung stehen, sodass die erste äußere Schicht durch in radialer Richtung des Schlauchs von den elastischen Fäden auf den Schlauch wirkende Kräfte verjüngt wird, wenn die erste äußere Schicht bzw. der Schlauch von dem Formkern entfernt wird.

Anschließend wird eine zweite äußere Schicht ebenfalls aus einem flächigen Bahnmaterial auf die elastischen Fäden aufgebracht und mit den elastischen Fäden verbunden. Daraufhin wird das auf diese Weise gebildete schlauchförmige Bahnmaterial in Längsrichtung aufgeschnitten, wobei lediglich die elastischen Fäden durchtrennt werden müssen, da die in eine Schlauchform gefaltete erste äußere Schicht und die zweite äußere Schicht so zueinander angeordnet werden, dass das schlauchförmige Bahnmaterial längs einer Linie aufgeschnitten werden kann, in der Endabschnitte der ersten äußeren Schicht sowie der zweiten äußeren Schicht jeweils aneinander anliegen. Auf diese Weise kann das schlauchförmige Bahnmaterial nach Durchtrennen der elastischen Fäden längs dieser Linie zu einem flächenelastischen Bahnmaterial aufgefalten und anschließend beispielsweise zu Unterbekleidung weiterverarbeitet werden.

Aus der DE 10 2010 013 288 A1 ist ein vergleichbares Verfahren bekannt, wobei die Vorspannung der elastischen Fäden dadurch erreicht wird, dass der Schlauch zunächst durch Verjüngung des Querschnitts des Formkerns verkleinert wird, die elastischen Fäden ohne Vorspannung um den verkleinerten Schlauch gewickelt und der Schlauch anschließend aufgespreitzt wird. Bei der Spreitzung werden auch die elastischen Fäden gespannt bis die Fäden vollständig an der Außenfläche der ersten äußeren Schicht bzw. des Schlauchs anliegen.

In der GB 1 250 788 A wird ein Hosenherstellungsverfahren beschrieben, bei dem Einschnitte in eine Materialbahn eingebracht werden, um Teile der Materialbahn nachträglich abtrennen zu können.

Bei dem aus dem Stand der Technik bekannten Verfahren wird zur Herstellung der Unterbekleidung zunächst das flächenelastische Bahnmaterial hergestellt und dieses anschließend in weiteren Verarbeitungsschritten auf bekannte Art und Weise zu der Unterbekleidung weiterverarbeitet.

Als Aufgabe der Erfindung wird es angesehen, die aus dem Stand der Technik bekannte Herstellung elastischer Hosen aus einem flächenelastischen Material so weiterzuentwickeln, dass die elastischen Hosen in einem einzigen Herstellungsprozess einfach und kostengünstig hergestellt werden können.

Diese Aufgabe wird erfindungsgemäß dadurchgelöst, dass in Transportrichtung nachfolgend das Hosenbahnmaterial in regelmäßigen Abständen längs eines Verbindungsabschnitts verschlossen wird, sodass durch den Verbindungsabschnitt in Querrichtung einander gegenüberliegend zwei näherungsweise gleich große Öffnungsabschnitte gebildet werden, und wobei in Transportrichtung nachfolgend das Hosenbahnmaterial beabstandet zu den Verbindungsabschnitten durchtrennt wird, wodurch eine elastische Hose gebildet wird.

Bei der Herstellung der elastischen Hose wird auf das aus dem Stand der Technik bekannte und eingangs beschriebene Verfahren zur Herstellung flächenelastischer Materialien zurückgegriffen, wobei im Unterschied zu diesem Verfahren die erste äußere Schicht beispielsweise durch Schweißen, eine Klebeverbindung oder dergleichen verschlossen wird, während bei dem aus dem Stand der Technik bekannten Verfahren das elastische Bahnmaterial lediglich zu dem Schlauch gefalten wird, wobei die Endabschnitte des elastischen Bahnmaterials aneinander anliegen und nicht miteinander verbunden werden. Zum Verschließen des Schlauchs kann der Schlauch entweder so gebildet werden, dass sich die Endabschnitte des aufgefalteten Bahnmaterials einander überlappen und die sich überlappenden Endabschnitte in dem Überlappungsbereich miteinander verbunden werden oder auch dadurch hergestellt werden, dass das Bahnmaterial so gefalten wird, dass Seitenkanten des gefalteten Bahnmaterials aneinander anliegen und die an die Seitenkanten angrenzenden Endabschnitte beispielsweise durch eine Naht oder dergleichen miteinander verbunden werden. Erfindungsgemäß ist vorgesehen, dass auch die zweite äußere Schicht auf vergleichbare Weise verschlossen werden kann.

Bei dem Bahnmaterial handelt es sich vorteilhafterweise um ein Faservliesmaterial, ein Webmaterial, eine Folie oder um ein mehrlagiges Laminat aus diesen oder vergleichbaren Materialien. Die zur Herstellung der Schlaucharmierung verwendeten elastischen Fäden können erfindungsgemäß unter anderem aus Elasthan, Gummi, synthetischem Gummi oder vergleichbaren Materialien hergestellt sein.

Bei der Anordnung der elastischen Fäden an der ersten äußeren Schicht kann entweder auf das aus dem Stand der Technik bekannte Verfahren zurückgegriffen werden, bei dem die erste äußere Schicht in eine konvexe Form gebracht wird und die elastischen Fäden unter Vorspannung auf die erste äußere Schicht aufgebracht werden. Es ist aber auch möglich, die erste äußere Schicht zu verjüngen und die elastischen Fäden auf die verjüngte äußere Schicht unter keiner oder einer geringeren Vorspannung aufzubringen.

Erfindungsgemäß wird die zweite äußere Schicht ebenfalls aus einem flächigen Bahnmaterial hergestellt, wobei vergleichbare Materialien verwendet werden können, die auch zur Herstellung der ersten äußeren Schicht verwendet werden.

Die Herstellung der Hosen aus dem Hosenbahnmaterial erfolgt dadurch, dass das Hosenbahnmaterial in regelmäßigen Abständen längs des Verbindungsabschnitts verschlossen werden. Das Verschließen längs des Verbindungsabschnitts kann erfindungsgemäß durch Schweißen, Kleben oder dergleichen erfolgen. Dabei werden Innenflächen der ersten äußeren Schicht in dem Verbindungsabschnitt miteinander verbunden.

Die Verbindung in dem Verbindungsabschnitt ist zweckmäßigerweise linienförmig ausgestaltet und weist vorteilhafterweise eine gebogene Kontur auf. Beim Tragen der mithilfe des erfindungsgemäßen Verfahrens hergestellten Hose liegt die Verbindung im Schritt der die Hose tragenden Person an, sodass die bogenförmige Kontur erfindungsgemäß an die Körperkontur in diesem Bereich angepasst ist.

Durch die Verbindung in dem Verbindungsabschnitt wird das Hosenbahnmaterial in Querrichtung, das heißt quer zur Transportrichtung des Hosenbahnmaterials so verschlossen, dass das schlauchförmige Hosenbahnmaterial in dem Abschnitt, in dem der Verbindungsabschnitt angeordnet ist durch die Verbindung in zwei schlauchförmige Bereiche unterteilt wird.

Zur Herstellung der Hose wird das in dem Verbindungsabschnitt verschlossene Hosenbahnmaterial beabstandet zu dem Verbindungsabschnitt durchtrennt, wodurch die elastische Hose gebildet wird. Das Durchtrennen kann durch einen konturierten und an eine Körperform angepassten Schnitt erfolgen, um unterschiedliche Hosenmodelle wie beispielsweise Slips oder Boxershorts herstellen zu können. Dadurch, dass das Hosenbahnmaterial in regelmäßigen Abständen durchtrennt wird, werden im Verlauf des Herstellungsverfahrens stetig Hosen gefertigt.

Um die Herstellung zu vereinfachen und Material einzusparen, ist erfindungsgemäß vorgesehen, dass das Bahnmaterial zwischen zwei spiegelbildlich ausgestalteten Verbindungsabschnitten, die beabstandet zueinander hergestellt werden, durchtrennt wird, sodass jeweils zwei paarweise spiegelbildlich ausgestaltete Hosen hergestellt werden.

Um die erste äußere Schicht, die elastischen Fäden und die zweite äußere Schicht miteinander zu verbinden, ist erfindungsgemäß vorgesehen, dass auf die elastischen Fäden ein Haftkleber aufgebracht wird. Es ist aber auch möglich und erfindungsgemäß vorgesehen, die zweite äußere Schicht mit der ersten äußeren Schicht beispielsweise durch Schweißen oder dergleichen miteinander zu verbinden, wobei die hierfür verwendeten Verbindungsstellen so ausgestaltet werden, dass die elastische Wirkung der elastischen Fäden nicht beeinträchtigt wird. Erfindungsgemäß können die verschiedenen Verbindungsmöglichkeiten auch kombiniert werden, sodass beispielsweise zusätzlich zu einer klebenden Verbindung eine Schweißverbindung verwendet werden kann.

Vorteilhafterweise ist erfindungsgemäß vorgesehen, dass das Hosenbahnmaterial vor der Herstellung des Verbindungsabschnitts fortlaufend aufgespreizt und in eine flache Form gezogen wird. Auf diese Weise kann der Verbindungsabschnitt besonders einfach beispielsweise durch Schweißen hergestellt werden. Der Verbindungsabschnitt wird bei dem in die flache Form gezogenen Hosenbahnmaterial in einem mittleren Bereich zwischen zwei in Längsrichtung des Hosenbahnmaterials verlaufenden Längsseiten des Hosenbahnmaterials angeordnet.

Bei einer weiteren vorteilhaften Ausgestaltung des erfindungsgemäßen Verfahrens ist vorteilhafterweise vorgesehen, dass die erste äußere Schicht und die elastischen Fäden vor dem Aufbringen der zweiten äußeren Schicht fortlaufend aufgespreizt und in eine flache Form gezogen werden. Auf diese Weise kann die Anordnung der zweiten äußeren Schicht an den elastischen Fäden erheblich vereinfacht werden.

Um die zweite äußere Schicht einfach an der in die flache Form gezogenen ersten äußeren Schicht anordnen zu können, ist erfindungsgemäß vorgesehen, dass die zweite äußere Schicht aus zwei Teilschichten hergestellt wird, wobei die beiden Teilschichten auf einander gegenüberliegenden Seiten der aufgespreizten ersten äußeren Schicht und der elastischen Fäden aufgebracht werden. Die Teilschichten können aus einem elastischen Bahnmaterial durch Auftrennen des elastischen Bahnmaterials oder durch Auffalten des elastischen Bahnmaterials längs einer mittig zwischen Längsseiten des elastischen Bahnmaterials verlaufenden Mittelinie hergestellt werden. Es ist aber auch möglich und erfindungsgemäß vorgesehen, dass unterschiedlichen Bahnen eines Bahnmaterials für die Herstellung der Teilschichten verwendet werden können, wobei vorteilhafterweise auch unterschiedliche Bahnmaterialien verwendet werden können, um beispielsweise unterschiedliche Eigenschaften einer Hosenvorderseite und einer Hosenrückseite zu ermöglichen.

Um die Eigenschaften der elastischen Hose weiter zu beeinflussen, ist erfindungsgemäß vorgesehen, dass auf die zweite äußere Schicht ebenfalls elastische Fäden in Form einer Schlaucharmierung aufgebracht werden und auf diese elastischen Fäden eine dritte äußere Schicht aufgebracht wird. Die dritte äußere Schicht und die elastischen Fäden können wiederum aus den gleichen Materialien hergestellt sein, wie die erste und zweite äußere Schicht sowie die bereits verwendeten und auf die erste äußere Schicht aufgebrachten elastischen Fäden. Die erste äußere Schicht, die zweite äußere Schicht, die dritte äußere Schicht sowie die zwischen den Schichten angeordneten elastischen Fäden stellen das Hosenbahnmaterial dar. Die dritte äußere Schicht kann erfindungsgemäß auf die gleiche Weise verarbeitet werden, wie die erste und die zweite äußere Schicht.

Das erfindungsgemäße Verfahren eignet sich auch zur Herstellung von Höschenwindeln oder dergleichen. Insbesondere hierzu ist erfindungsgemäß vorgesehen, dass zwischen der ersten äußeren Schicht und der zweiten äußeren Schicht und/oder zwischen der zweiten äußeren Schicht und der dritten äußeren Schicht ein Granulat und/oder ein Fasermaterial angeordnet werden. Bei dem Granulat kann es sich erfindungsgemäß um einen Superabsorber handeln, der eine vergleichbare große Menge Flüssigkeit aufnehmen kann.

Erfindungsgemäß ist insbesondere für die Herstellung von Höschenwindeln vorgesehen, dass die erste äußere Schicht und/oder die zweite äußere Schicht und/oder die dritte äußere Schicht aus einem hydrophoben oder aus einem hydrophilen Material hergestellt sind. Auf diese Weise können die Eigenschaften der Höschenwindeln beeinflusst werden, da durch eine geeignete Auswahl des zur Herstellung der Schichten verwendeten Materials erreicht werden kann, dass Feuchtigkeit von der die Höschenwindel tragenden Person schnell von deren Körper in Richtung der zweiten äußeren Schicht bzw. der dritten äußeren Schicht transportiert wird, wobei gleichzeitig vermieden werden kann, dass Feuchtigkeit aus der zweiten äußeren Schicht oder der dritten äußeren Schicht austritt bzw. zurück zur ersten äußeren Schicht und durch die erste äußere Schicht hindurch transportiert wird.

Um eine ausreichende Flächenelastizität der hergestellten Hose zu erreichen, ist erfindungsgemäß vorgesehen, dass eine Länge der elastischen Fäden 120% bis 500% der Länge der elastischen Fäden im ungespannten Zustand entspricht, wenn die erste äußere Schicht eine konvexe Form aufweist. Die konvexe Form im Sinne dieser Erfindung ist dadurch gekennzeichnet, dass die erste äußere Schicht vollständig aufgespreizt ist, sodass sämtliche Verbindungslinien zweier Punkte eines in Querrichtung der ersten äußeren Schicht verlaufenden Umfangs vollständig innerhalb dieses Umfangs verlaufen.

Um die Flächenelastizität der hergestellten Hose bereichsweise beeinflussen zu können, ist erfindungsgemäß vorgesehen, dass mindestens ein elastischer Faden aus einem anderen Material besteht als die übrigen elastischen Fäden. Beispielsweise können Fäden unterschiedlicher Elastizität verwendet werden, wobei einige Fäden zur Verbesserung der Stabilität und Langlebigkeit der hergestellten Hose vorgesehen sein können sowie andere Fäden dazu dienen können, die erforderliche Flächenelastizität bereitzustellen.

Zur Herstellung der schlauchförmigen ersten äußeren Schicht ist erfindungsgemäß vorgesehen, dass das flächige Bahnmaterial in der Längsrichtung des Bahnmaterials fortlaufend über einen länglichen Formkern zu dem Schlauch gefalten wird, wobei der Formkern so ausgestaltet ist, dass der Schlauch eine konvexe Form aufweist.

Erfindungsgemäß ist vorteilhafterweise vorgesehen, dass eine Form der zweiten äußeren Schicht und/oder der dritten äußeren Schicht an die konvexe Form der ersten äußeren Schicht angepasst ist und so auf die elastischen Fäden aufgebracht wird. Erfindungsgemäß können die zweite äußere Schicht und/oder die dritte äußere Schicht über die über den Formkern gefaltene erste äußere Schicht und die auf der ersten äußeren Schicht angeordneten elastischen Fäden in vergleichbarer Weise in eine Schlauchform gefalten werden.

Vorteilhafterweise ist erfindungsgemäß vorgesehen, dass die elastischen Fäden unter Vorspannung auf die erste äußere Schicht und/oder die zweite äußere Schicht aufgebracht werden.

Um die elastischen Fäden auf die erste äußere Schicht aufbringen zu können, ist erfindungsgemäß vorgesehen, dass die elastischen Fäden auf Fadenspulen aufgewickelt sind, wobei die Fadenspulen an mindestens einem Planetenrad angeordnet sind, wobei die erste äußere Schicht in der konvexen Form durch das Planetenrad hindurchgeführt wird, wobei das Planetenrad um die erste äußere Schicht in eine Rotationsbewegung versetzt wird und wobei die elastischen Fäden fortlaufend von den Fadenspulen abgewickelt werden und auf die erste äußere Schicht aufgebracht werden. Auf analoge Weise können auch elastische Fäden auf die zweite äußere Schicht aufgebracht werden.

Eine Mittelachse des Planetenrads kann erfindungsgemäß axial zu einer in Transportrichtung verlaufenden Mittelachse der schlauchförmig aufgefalteten ersten äußeren Schicht verlaufen. Um den Verlauf bzw. die Ausrichtung der elastischen Fäden an der ersten äußeren Schicht zu beeinflussen, ist es aber auch möglich und erfindungsgemäß vorgesehen, dass die Mittelachse des Planetenrads und die Mittelachse der ersten äußeren Schicht einen Winkel zueinander aufweisen. Auf diese Weise ist es erfindungsgemäß möglich, dass die elastischen Fäden bereichsweise in der Querrichtung auf der ersten äußeren Schicht verlaufen. Auf analoge Weise können auch die zur Anordnung der elastischen Fäden auf der zweiten äußeren Schicht verwendeten Planetenräder ausgerichtet sein.

Zur Beeinflussung der Eigenschaften der elastischen Hose und insbesondere zur Beeinflussung der Flächenelastizität der Hose ist erfindungsgemäß vorgesehen, dass sich Abrollgeschwindigkeiten beim Abrollen der elastischen Fäden von unterschiedlichen Fadenspulen voneinander unterscheiden.

Weitere vorteilhafte Ausgestaltungen des erfindungsgemäßen Verfahrens werden anhand von in der Zeichnung dargestellten Ausführungsbeispielen näher erläutert. Es zeigt:
Figur 1 eine schematisch dargestellte Ansicht wesentlicher Funktionen und Verfahrensschritte bei der Durchführung des erfindungsgemäßen Verfahrens zur Herstellung einer Hose,
Figur 2 eine schematische Darstellung eines alternativ ausgestalteten Verfahrens zur Herstellung einer elastischen Hose, wobei zwischen die zweite äußere Schicht und die dritte äußere Schicht ein Superabsorber eingebracht wird,
Figuren 3 und 4 schematische Darstellungen der mithilfe des erfindungsgemäßen Verfahrens hergestellten elastischen Hosen,
Figur 5 eine schematische Darstellung mit dem erfindungsgemäßen Verfahren hergestellter Hosen, wobei spiegelbildlich ausgestaltete Verbindungsabschnitte benachbart zueinander angeordnet wurden und auf diese Weise paarweise spiegelbildlich ausgestaltete elastische Hosen hergestellt wurden,
Figur 6 eine schematische Darstellung eines Planetenrads, dessen Mittelachse einen Winkel zur Mittelachse der ersten äußeren Schicht aufweist,
Figur 7 ein schematisch dargestellter Verlauf der mithilfe der in Figur 6 dargestellten Anordnung an der äußeren Schicht aufgebrachten elastischen Fäden.

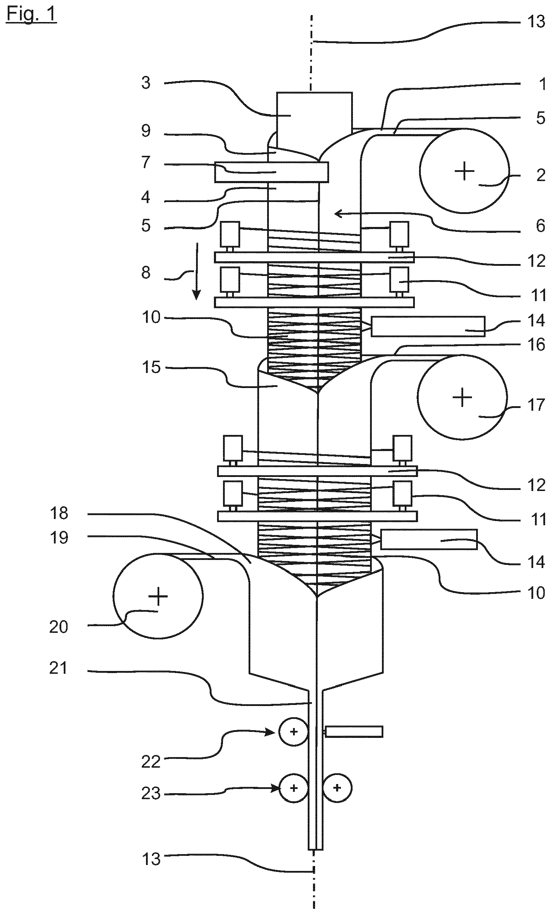

Figur 1 zeigt eine schematische Darstellung des Ablaufs des erfindungsgemäßen Verfahrens. Zunächst wird ein flächiges Bahnmaterial 1 von einer Materialrolle 2 fortlaufend abgerollt und über einen Formkern 3 in eine konvexe Form zu einem Schlauch 4 gefalten. Dabei wird das Bahnmaterial 1 so gefalten, dass zu Seitenkanten 5 benachbarte Endabschnitte 6 des Bahnmaterials einander überlappend angeordnet sind. In der Darstellung ist lediglich eine Seitenkante 5 und ein Endabschnitt 6 dargestellt, da sich die zweite Seitenkante und der entsprechende zweite Endabschnitt unterhalb des ersten Endabschnitts 6 befinden und daher nicht sichtbar sind.

Nach dem Falten des flächigen Bahnmaterials 1 zu einem Schlauch 4 wird der Schlauch 4 mittels einer Schweißeinrichtung 7 im Bereich der Endabschnitte 6 in Längsrichtung 8 verschlossen. Der verschlossene Schlauch 4 bildet eine erste äußere Schicht 9. In Längsrichtung bzw. Transportrichtung 8 nachfolgend wird die erste äußere Schicht 9 mit elastischen Fäden 10 in Form einer Schlaucharmierung umwickelt. Die elastischen Fäden 10 werden von Fadenspulen 11 abgewickelt, die an Planetenrädern 12 angeordnet sind. Die Planetenräder 12 werden zu diesem Zweck in eine Rotationsbewegung um die erste äußere Schicht 9 bzw. den Formkern 3 versetzt. Eine Mittelachse bzw. Rotationsachse der Planetenräder entspricht einer Mittelachse 13 der ersten äußeren Schicht 9.

Im Anschluss wird mithilfe einer Haftklebereinrichtung 14 Haftkleber auf die elastischen Fäden 10 aufgebracht. In Transportrichtung 8 nachfolgend wird dann eine zweite äußere Schicht 15 auf die elastischen Fäden 10 aufgebracht, wobei die zweite äußere Schicht 15 ebenfalls aus einem Bahnmaterial 16 besteht, dass von einer Materialrolle 17 abgerollt wird.

In dieser Darstellung wird in Transportrichtung 8 nachfolgend die zweite äußere Schicht 15 erneut mit elastischen Fäden 10 umwickelt, die elastischen Fäden 10 mit Haftkleber besprüht und anschließend eine dritte äußere Schicht 18 auf die elastischen Fäden 10 aufgebracht. Die dritte äußere Schicht 18 wird wiederum aus einem Bahnmaterial 19 hergestellt, das von einer Materialrolle 20 abgerollt wird.

Anschließend wird das auf diese Weise hergestellte Hosenbahnmaterial 21 in eine flache Form gezogen und in regelmäßigen Abständen jeweils längs eines Verbindungsabschnitts mithilfe einer Schweißeinrichtung 22 verschlossen und anschließend beabstandet zu dem Verbindungsabschnitt mithilfe einer Schneideinrichtung 23 durchtrennt, wodurch stetig elastische Hosen hergestellt werden.

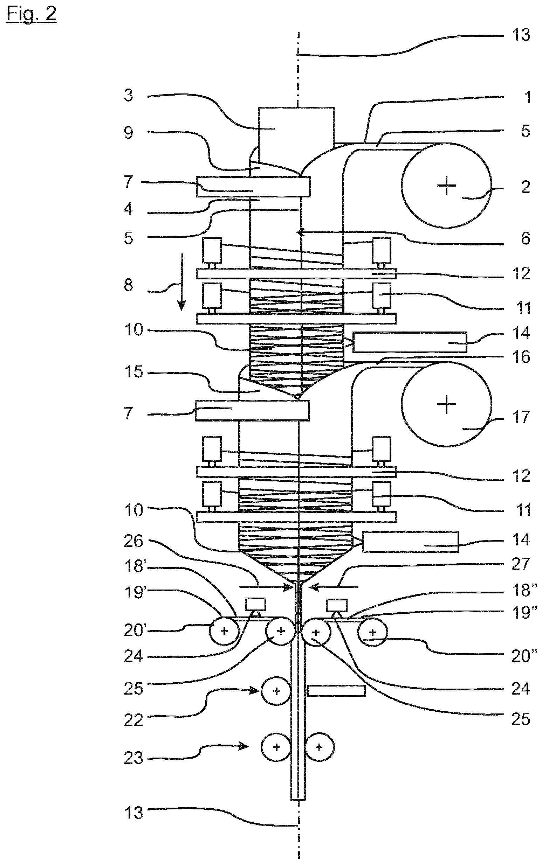

Bei dem in Figur 2 schematisch dargestellten Ablauf des erfindungsgemäßen Verfahrens werden die erste äußere Schicht 9 und die zweite äußere Schicht 15 vor Aufbringen der dritten äußeren Schicht 18 in die flache Form gezogen. Bei dem Aufbringen der dritten äußeren Schicht 18, die in diesem Ausführungsbeispiel aus zwei Teilschichten 18' und 18" besteht, die jeweils aus separaten Bahnmaterialien 19' und 19" hergestellt sind und jeweils von Materialrollen 20' und 20" abgerollt werden, wird ein Superabsorber 24 auf die Teilschichten 18' und 18" aufgebracht und mithilfe von Vakuumrollen 25 an den Teilschichten 18' und 18" gehalten bis die Teilschichten 18' und 18" auf einander gegenüberliegenden Seiten 26 und 27 des in die flache Form gezogenen Schlauchs 4 aufgebracht werden. Der weitere Ablauf des Herstellungsverfahrens entspricht dem in Figur 1 dargestellten Ablauf.

Bei den in Figur 1 und Figur 2 schematisch dargestellten Verfahrensabläufen kann auch vorgesehen sein, dass lediglich die zweite äußere Schicht 15 aufgebracht wird und auf die dritte äußere Schicht 18 verzichtet wird, wobei in dem in Figur 2 dargestellten Ausführungsbeispiel der Superabsorber 24 auf entsprechende Teilschichten der zweiten äußeren Schicht 15 aufgebracht werden könnten.

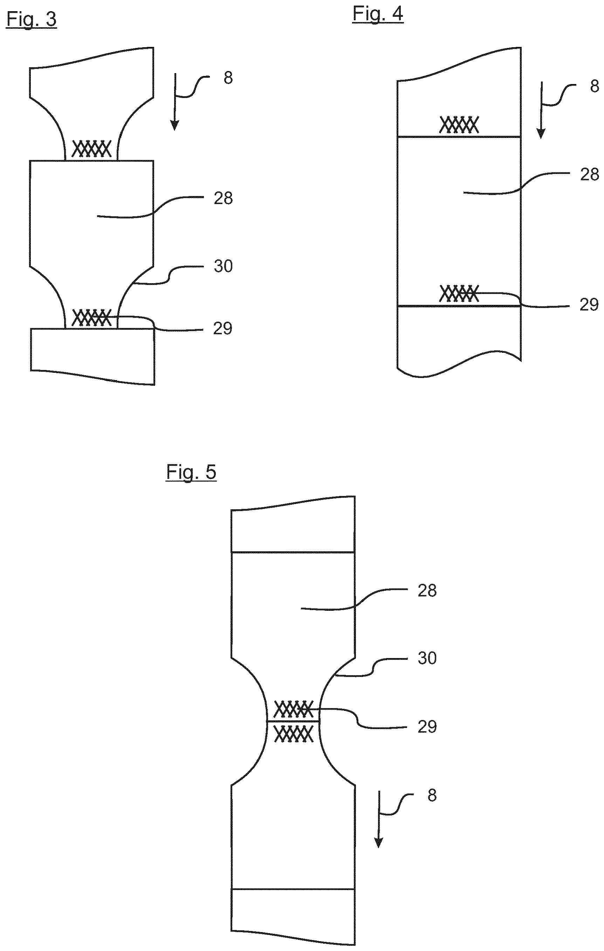

Figuren 3 und 4 zeigen schematische Draufsichten auf mehrere, mithilfe des erfindungsgemäßen Verfahrens hergestellte Hosen 28. Die Hosen 28 wurden jeweils in einem Verbindungsabschnitt 29 durch Verschweißen verschlossen und anschließend längs einer Schneidkontur 30 aufgetrennt. Bei den in der Figur 3 dargestellten Hosen 28 wurde die Schneidkontur 30 an eine Körperform angepasst, während bei den in der Figur 4 dargestellten Hosen 28 ein einfacher Schnitt quer zur Längsachse 8 gewählt wurde.

In Figur 5 sind schematisch alternativ hergestellte elastische Hosen 28 dargestellt, wobei bei der Herstellung das Hosenbahnmaterial 21 in benachbart zueinander angeordneten Verbindungsabschnitten 29 miteinander verschweißt wurde und anschließend längs einer Schneidkontur 30 durchtrennt wurde, wodurch paarweise spiegelbildlich angeordnete Hosen 28 hergestellt wurden.

Erfindungsgemäß werden die durch das Verfahren hergestellten Hosen 28 in weiteren Verarbeitungsschritten nachbearbeitet und beispielsweise mit geeigneten Nähten im Bereich der vorgenommenen Schnitte versehen.

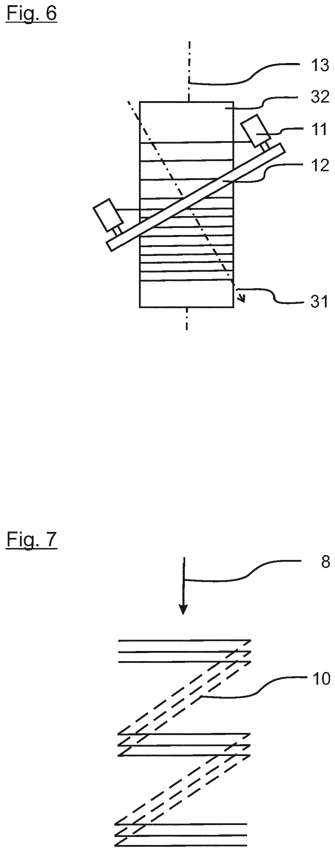

In Figur 6 ist eine alternative Anordnung eines Planetenrads 12 dargestellt, wobei eine Mittelachse 31 des Planetenrads 12 einen Winkel zu einer Mittelachse 13 einer zu umwickelnden und in eine konvexe Form gezogenen äußeren Schicht 32 aufweist. Auf diese Weise kann beispielsweise das in Figur 7 schematisch dargestellte Wickelmuster bzw. der dort dargestellte Verlauf der elastischen Fäden 10 um die äußere Schicht 32 hergestellt werden. Die elastischen Fäden 10 verlaufen in einem durchgezogen dargestellten Abschnitt quer zur Längsrichtung 8 der äußeren Schicht 32 und in einem gestrichelt dargestellten zweiten Abschnitt schräg zu der Längsrichtung 8.

## Patentansprüche

1. Verfahren zur kontinuierlichen Herstellung elastischer Hosen (28), wobei ein flächiges Bahnmaterial (1, 16, 19) in einer Längsrichtung (8) des Bahnmaterials (1, 16, 19) fortlaufend transportiert, zu einem geschlossenen Schlauch (4) geformt und in Längsrichtung (8) verlaufend verschlossen wird, wobei der verschlossene Schlauch (4) eine erste äußere Schicht (9) bildet und wobei die erste äußere Schicht (9) in Transportrichtung (8) nachfolgend mit elastischen Fäden (10) in Form einer Schlaucharmierung umwickelt wird, sodass die elastischen Fäden (10) gespannt sind, wenn die äußere Schicht (9) eine konvexe Form aufweist, wobei auf die elastischen Fäden (10) in Transportrichtung (8) nachfolgend fortlaufend eine zweite äußere Schicht (15) aufgebracht und mit den elastischen Fäden (10) und/oder der ersten äußeren Schicht (9) verbunden wird, wobei die erste äußere Schicht (9), die elastischen Fäden (10) und die zweite äußere Schicht (15) ein elastisches Hosenbahnmaterial (21) bilden, **dadurch gekennzeichnet, dass** in Transportrichtung (8) nachfolgend das Hosenbahnmaterial (21) in regelmäßigen Abständen längs eines Verbindungsabschnitts (29) verschlossen wird, sodass durch den Verbindungsabschnitt (29) in Querrichtung einander gegenüberliegend zwei näherungsweise gleich große Öffnungsabschnitte gebildet werden, und wobei in Transportrichtung (8) nachfolgend das Hosenbahnmaterial (21) beabstandet zu dem Verbindungsabschnitt (29) durchtrennt wird, wodurch eine elastische Hose (28) gebildet wird.

2. Verfahren gemäß Anspruch 1, **dadurch gekennzeichnet, dass** auf die elastischen Fäden (10) ein Haftkleber aufgebracht wird.

3. Verfahren gemäß Anspruch 1 oder Anspruch 2, **dadurch gekennzeichnet, dass** das Hosenbahnmaterial (21) vor der Herstellung des Verbindungsabschnitts (29) fortlaufend aufgespreizt und in eine flache Form gezogen wird.

4. Verfahren gemäß Anspruch 1 oder Anspruch 2, **dadurch gekennzeichnet, dass** die erste äußere Schicht (9) und die elastischen Fäden (10) vor dem Aufbringen der zweiten äußeren Schicht (15) fortlaufend aufgespreizt und in eine flache Form gezogen werden.

5. Verfahren gemäß Anspruch 4, **dadurch gekennzeichnet, dass** die zweite äußere Schicht (15) aus zwei Teilschichten hergestellt wird, wobei die beiden Teilschichten auf einander gegenüberliegenden Seiten (26, 27) der aufgespreizten ersten äußeren Schicht (9) und der elastischen Fäden (10) aufgebracht werden.

6. Verfahren gemäß einem der voranstehenden Ansprüche, **dadurch gekennzeichnet, dass** auf die zweite äußere Schicht (15) ebenfalls elastische Fäden (10) in Form einer Schlaucharmierung aufgebracht werden und auf diese elastischen Fäden (10) eine dritte äußere Schicht (18) aufgebracht wird.

7. Verfahren gemäß einem der voranstehenden Ansprüche, **dadurch gekennzeichnet, dass** zwischen der ersten äußeren Schicht (9) und der zweiten äußeren Schicht (15) und/oder zwischen der zweiten äußeren Schicht (15) und der dritten äußeren Schicht (18) ein Granulat und/oder ein Fasermaterial angeordnet werden.

8. Verfahren gemäß einem der voranstehenden Ansprüche, **dadurch gekennzeichnet, dass** eine Länge der elastischen Fäden (10) 120% bis 500% der Länge der elastischen Fäden (10) im ungespannten Zustand entspricht, wenn die erste äußere Schicht (9) eine konvexe Form aufweist.

9. Verfahren gemäß einem der voranstehenden Ansprüche, **dadurch gekennzeichnet, dass** mindestens ein elastischer Faden (10) aus einem anderen Material besteht als die übrigen elastischen Fäden.

10. Verfahren gemäß einem der voranstehenden Ansprüche, **dadurch gekennzeichnet, dass** das flächige Bahnmaterial (1, 16, 19) in der Längsrichtung (8) des Bahnmaterials (1, 16, 19) fortlaufend über einen länglichen Formkern (3) zu dem geschlossenen Schlauch (4) gefalten wird, wobei der Formkern (3) so ausgestaltet ist, dass der Schlauch (4) eine konvexe Form aufweist.

11. Verfahren gemäß Anspruch 10, **dadurch gekennzeichnet, dass** eine Form der zweiten äußeren Schicht (15) und/oder der dritten äußeren Schicht (18) an die konvexe Form der ersten äußeren Schicht (9) angepasst ist und so auf die elastischen Fäden (10) aufgebracht wird.

12. Verfahren gemäß Anspruch 10 oder Anspruch 11, **dadurch gekennzeichnet, dass** die elastischen Fäden (10) unter Vorspannung auf die erste äußere Schicht (9) und/oder die zweite äußere Schicht (15) aufgebracht werden.

13. Verfahren gemäß einem der Ansprüche 10 bis 12, **dadurch gekennzeichnet, dass** die elastischen Fäden (10) auf Fadenspulen (11) aufgewickelt sind, wobei die Fadenspulen (11) an mindestens einem Planetenrad (12) angeordnet sind, wobei die erste äußere Schicht (9) in der konvexen Form durch das Planetenrad (12) hindurchgeführt wird, wobei das Planetenrad (12) um die erste äußere Schicht (9) in eine Rotationsbewegung versetzt wird und wobei die elastischen Fäden (10) fortlaufend von den Fadenspulen (11) abgewickelt werden und auf die erste äußere Schicht (9) aufgebracht werden.

14. Verfahren gemäß Anspruch 13, **dadurch gekennzeichnet, dass** das Planetenrad (12) quer zur Längsrichtung (8) der ersten äußeren Schicht (9) ausgerichtet ist.

15. Verfahren gemäß Anspruch 13 oder Anspruch 14, **dadurch gekennzeichnet, dass** sich Abrollgeschwindigkeiten beim Abrollen der elastischen Fäden (10) von unterschiedlichen Fadenspulen (11) voneinander unterscheiden.

## Claims

1. Method for continuously manufacturing elastic pants (28), wherein a plan web material (1, 16, 19) is continuously transported in a longitudinal direction (8) of the web material (1, 16, 19), is formed into a closed tube (4) and is closed in the longitudinal direction (8), wherein the closed tube (4) forms a first outer layer (9) and wherein the first outer layer (9) is subsequently wound around with elastic threads (10) in the transport direction (8) in the form of a tubular reinforcement, so that the elastic threads (10) are taut when the outer layer (9) has a convex shape, wherein a second outer layer (15) is subsequently and continuously applied to the elastic threads (10) in the transport direction (8) and connected to the elastic threads (10) and/or the first outer layer (9), wherein the first outer layer (9), the elastic threads (10) and the second outer layer (15) form an elastic web material (21) for pants, **characterized in that** subsequently the web material (21) for pants is closed at regular intervals along a joining section (29), so that two opening sections of approximately the same size are formed by the joining section (29) in transverse direction as to oppose one another, and wherein subsequently the web material (21) for pants is cut at a distance to the joining section (29) in the transport direction (8), thereby forming elastic pants (28) .

2. Method according to claim 1, **characterized in that** an adhesive is applied to the elastic threads (10).

3. Method according to claim 1 or claim 2, **characterized in that** the web material (21) for pants is continuously spread and pulled in a flat shape before producing the joining section (29) .

4. Method according to claim 1 or claim 2, **characterized in that** the first outer layer (9) und the elastic threads (10) are continuously spread and pulled in a flat shape before applying the second outer layer (15).

5. Method according to claim 4, **characterized in that** the second outer layer (15) is produced from two sub-layers, wherein the two sub-layers are applied to opposite sides (26, 27) of the spread first outer layer (9) and the elastic threads (10).

6. Method according to one of the preceding claims, **characterized in that** elastic threads (10) are also applied to the second outer layer (15) in the form of a tubular reinforcement and a third outer layer (18) is applied to these elastic threads (10).

7. Method according to one of the preceding claims, **characterized in that** a granular material and/or a fibrous material is arranged between the first outer layer (9) and the second outer layer (15) and/or between the second outer layer (15) and the third outer layer (18).

8. Method according to one of the preceding claims, **characterized in that** a length of the elastic threads (10) corresponds to 120% to 500% of the length of the elastic threads (10) in a non-taut state, when the first outer layer (9) has a convex shape.

9. Method according to one of the preceding claims, **characterized in that** at least one elastic thread (10) consists of a material different from that of the remaining elastic threads.

10. Method according to one of the preceding claims, **characterized in that** the plan web material (1, 16, 19) is continuously folded in the longitudinal direction (8) of the plan web material (1, 16, 19) into the closed tube (4) over an elongated shaping core (3), wherein the shaping core (3) is designed in such a way that the tube (4) has a convex shape.

11. Method according to claim 10, **characterized in that** a shape of the second outer layer (15) and/or the third outer layer (18) is adapted to the convex shape of the first outer layer (9) and is thus applied to the elastic threads (10).

12. Method according to claim 10 or claim 11, **characterized in that** the elastic threads (10) are applied to the first outer layer (9) and/or the second outer layer (15) under prestress.

13. Method according to one of claims 10 to 12, **characterized in that** the elastic threads (10) are wound on thread bobbins (11), wherein the thread bobbins (11) are arranged on at least one planetary gear (12), wherein the first outer layer (9) is guided through the planetary gear (12) in the convex shape, wherein the planetary gear (12) is caused to rotate around the first outer layer (9) and wherein the elastic threads (10) are continuously un-wound from the thread bobbins (11) and applied to the first outer layer (9).

14. Method according to claim 13, **characterized in that** the planetary gear (12) is oriented transversely to the longitudinal direction (8) of the first outer layer (9).

15. Method according to claim 13 or claim 14, **characterized in that** un-winding speeds differ from one another when un-winding the elastic threads (10) from different thread bobbins (11).

## Revendications

1. Procédé de fabrication continue de culottes (28) élastiques, une matière plate en bande (1, 16, 19) étant transportée en continu dans un sens longitudinal (8) de la matière en bande (1, 16, 19), formée en un boyau (4) fermé et refermée en se déplaçant dans le sens longitudinal (8), le boyau (4) fermé formant une première couche extérieure (9) et la première couche extérieure (9), en suivant dans le sens de transport (8), étant enroulée avec des fils élastiques (10) en forme d'un renforcement de boyau, de sorte que les fils élastiques (10) sont tendus lorsque la couche extérieure (9) présente une forme convexe, une deuxième couche extérieure (15) étant appliquée sur les fils élastiques (10), en suivant en continu dans le sens de transport (8), et reliée aux fils élastiques (10) et/ou à la première couche extérieure (9), la première couche extérieure (9), les fils élastiques (10) et la deuxième couche extérieure (15) formant un matière élastique en bande pour culottes (21), **caractérisé en ce que**, en suivant dans le sens de transport (8), la matière en bande pour culottes (21) est fermée à intervalles réguliers le long d'une section de raccordement (29), de sorte que deux sections d'ouverture approximativement de même taille sont formées par la section de raccordement (29) dans le sens transversal en étant situées à l'opposé l'une de l'autre, la matière en bande pour culottes (21), en suivant dans le sens de transport (8), étant coupée à distance de la section de raccordement (29), sur quoi une culotte élastique (28) est formée.

2. Procédé selon la revendication 1, **caractérisé en ce qu'**un adhésif est appliqué sur les fils élastiques (10).

3. Procédé selon la revendication 1 ou la revendication 2, **caractérisé en ce que** la matière en bande pour culottes (21), avant la fabrication de la section de raccordement (29), est écartée en continu et étirée en une forme plate.

4. Procédé selon la revendication 1 ou la revendication 2, **caractérisé en ce que** la première couche extérieure (9) et les fils élastiques (10) sont écartés en continu avant l'application de la deuxième couche extérieure (15) et sont étirés en une forme plate.

5. Procédé selon la revendication 4, **caractérisé en ce que** la deuxième couche extérieure (15) est fabriquée à partir de deux couches partielles, les deux couches partielles étant appliquées sur des côtés (26, 27) opposés l'un à l'autre de la première couche extérieure (9) écartée et des fils élastiques (10) .

6. Procédé selon l'une quelconque des revendications précédentes, **caractérisé en ce que** des fils élastique (10) en forme d'un renforcement de boyau sont également appliqués sur la deuxième couche extérieure (15) et **en ce que** sur ces fils élastiques (10) est appliquée une troisième couche extérieure (18) .

7. Procédé selon l'une quelconque des revendications précédentes, **caractérisé en ce qu'**un granulat et/ou une matière en fibres sont disposés entre la première couche extérieure (9) et/ou entre la deuxième couche extérieure (15) et la troisième couche extérieure (18).

8. Procédé selon l'une quelconque des revendications précédentes, **caractérisé en ce qu'**une longueur des fils élastiques (10) correspond à 120% à 500% de la longueur des fils élastiques (10) à l'état non tendu lorsque la première couche extérieure (9) présente une forme convexe.

9. Procédé selon l'une quelconque des revendications précédentes, **caractérisé en ce qu'**au moins un fil élastique (10) consiste en une autre matière que les autres fils élastiques.

10. Procédé selon l'une quelconque des revendications précédentes, **caractérisé en ce que** la matière en bande (1, 16, 19) plate est pliée dans le sens longitudinal (8) de la matière en bande (1, 16, 19) de manière continue sur un noyau de formage oblong (3) pour se transformer en boyau fermé (4), le noyau de formage (3) étant conçu de façon à ce que le boyau (4) présente une forme convexe.

11. Procédé selon la revendication 10, **caractérisé en ce qu'**une forme de la deuxième couche extérieure (15) et/ou de la troisième couche extérieure (18) est adaptée à la forme convexe de la première couche extérieure (9) et est ainsi appliquée sur les fils élastiques (10).

12. Procédé selon la revendication 10 ou la revendication 11, **caractérisé en ce que** les fils élastiques (10) sont appliqués sur la première couche extérieure (9) et/ou sur la deuxième couche extérieure (15) en étant précontraints.

13. Procédé selon l'une quelconque des revendications 10 à 12, **caractérisé en ce que** les fils élastiques (10) sont enroulés sur des bobines de fil (11), les bobines de fil (11) étant disposées sur au moins une roue planétaire (12), la première couche extérieure (9) étant guidée dans la forme convexe à travers la roue planétaire (12), la roue planétaire (12) étant mise dans un mouvement de rotation autour de la première couche extérieure (9) et les fils élastiques (10) étant déroulés en continu des bobines de fil (11) et appliqués sur la première couche extérieure (9).

14. Procédé selon la revendication 13, **caractérisé en ce que** la roue planétaire (12) est orientée transversalement au sens longitudinal (8) de la première couche extérieure (9).

15. Procédé selon la revendication 13 ou 14, **caractérisé en ce que** des vitesses de déroulement, lors du déroulement des fils élastiques (10) de différentes bobines de fil (11), se différencient les unes des autres.
